# EUROPEAN PATENT SPECIFICATION

(11) **EP 1 635 718 B1**
(45) Date of publication and mention of the grant of the patent: **24.02.2021**
(21) Application number: 04755466.2
(22) Date of filing: 16.06.2004
(51) Int. Cl.: A61B 17/32, A61B 17/00

(54) **HAND ACTIVATED ULTRASONIC INSTRUMENT**
VON HAND AKTIVIERTES ULTRASCHALLINSTRUMENT
INSTRUMENT A ULTRASONS A ACTIVATION MANUELLE

(30) Priority: 17.06.2003 US 478984 P
(43) Date of publication of application: 22.03.2006
(60) Divisional application: 10196993.9
(73) Proprietor: Ethicon LLC, 00969 Guaynabo (PR)
(72) Inventor: MUIR, Stephanie, J., Loveland, OH 45140 (US); PROCH, Francis, S., Pickerington, OH 43147 (US); DUTTA, Sudeep, N., Mainneville, OH 45039 (US); KRAMER, Kenneth, S., Loveland, OH 45140 (US)
(74) Representative: Tunstall, Christopher Stephen
(86) International application number: PCT/US2004/019313
(87) International publication number: WO 2004/112844

(56) References cited:
- WO-A-01/67970
- DE-U1- 20 021 619
- US-A- 4 662 068
- US-A- 5 873 873
- US-A- 5 873 873
- US-A- 5 938 633
- US-A- 5 989 274
- US-A- 6 056 735
- US-A1- 2002 057 541
- US-A1- 2002 107 538

## Description

### Reference to Related Applications

The present application claims the priority benefit of U.S. provisional patent application serial no. 60/478,984, filed on June 17, 2003.

This application contains subject matter related to co-owned patent application nos. 09/879,319 and 09/693,549.

### Field of the Invention

The present invention relates generally to ultrasonic surgical devices, and more particularly to an ultrasonic surgical clamp coagulator apparatus for coagulating and/or cutting tissue, including a hand activated switch positioned on the handle for easy access by the surgeon.

### Background of the Invention

Ultrasonic surgical instruments are finding increasingly widespread applications in surgical procedures by virtue of the unique performance characteristics of such instruments. Depending upon specific instrument configurations and operational parameters, ultrasonic surgical instruments can provide substantially simultaneous cutting of tissue and hemostasis by coagulation, desirably minimizing patient trauma. The cutting action is typically effected by an end-effector at the distal end of the instrument, with the end-effector transmitting ultrasonic energy to tissue brought into contact therewith. Ultrasonic instruments of this nature can be configured for open surgical use, or laparoscopic or endoscopic surgical procedures.

Ultrasonic surgical instruments have been developed that include a clamp mechanism to press tissue against the end-effector (i.e. the cutting blade) of the instrument in order to couple ultrasonic energy to the tissue of a patient. Such an arrangement (sometimes referred to as a clamp coagulator shears or an ultrasonic transactor) is disclosed in U.S. Pat. Nos. 5,322,055; 5,873,873 and 6,325,811. The surgeon activates the clamp pad to press against the end-effector by squeezing on the handgrip or handle.

A foot pedal operated by the surgeon while simultaneously applying pressure to the handle to press tissue between the clamp pad and end-effector activates a generator that provides energy that is transmitted to the cutting blade for cutting and coagulating tissue. Key drawbacks with this type of instrument activation include the loss of focus on the surgical field while the surgeon searches for the foot pedal the foot pedal getting in the way of the surgeon's movement during a procedure and surgeon leg fatigue during long cases.

US 5,873,873 and US 5,938,633 disclose ultrasonic surgical devices having a separate switch connected to a generator for activating ultrasonic energy. The switch may be foot or hand activated.

DE 200 21 619 and US 2002/0057541 each disclose a type of handheld surgical instrument comprising an activation switch about the handle. These instruments are substantially axial in form and are accordingly awkward and uncomfortable in use.

US 4,662,068 discloses a suture fusing and cutting apparatus having a scissor-type arrangement and a switch on one of the handles. The scissor arrangement allows for the jaws of the apparatus to be easily closed.

US 5,989,274 and WO 01/67970 each disclose an instrument having a pistol-grip like arrangement and a switch provided on the handle. In the case of WO 01/67970, the switch controls a motor which drives a rotatable waveguide.

US 6,056,735 discloses various ultrasound treatments systems, including a clamp coagulator with a treatment section and holding member positioned at the distal end of a sheath. The handle of the instrument includes a pressure switch for activating the ultrasound energy.

US 2002/0107538 discloses various ultrasonic operation systems including a clamp coagulator having a scissors type handpiece. A switch unit may be clipped to the handpiece to provide a hand-activated switch.

US 6056735 A relates to an ultrasound treatment system that comprises: ultrasonic transducers for generating ultrasonic vibrations; a handpiece including the ultrasonic transducers and serving as an operation unit; and a probe connected to the ultrasonic transducers and serving as a vibration conveyer for conveying ultrasonic transducers to a distal member constituting a stationary section of a treatment unit used to treat a living tissue.

US 2002/107538 A1 relates to an ultrasonic operation system that comprises: a transmission cable that is connected detachably, through a connector, to a connector receptacle in a main apparatus unit; plural types of transducers provided with a connector receptacle each capable of detachably connecting the connector of the transmission cable, an identification element for outputting its own identification signal, and a ultrasonic vibrator, correspondingly; and a main apparatus unit for receiving the identification signals in an identification circuit, automatically controlling drive signals that a control circuit causes a drive signal generator unit to generate, and automatically displaying the identified instrument on a control-display unit.

US 5938633 A relates to a surgical instrument that includes a transducer assembly adapted to vibrate at an ultrasonic frequency. An end effector is adapted to receive ultrasonic vibration from the transducer assembly and to propagate the ultrasonic vibration from a first end to a second end. The end effector is rotatable with respect to the handpiece assembly while the transducer assembly vibrates at the ultrasonic frequency.

The present invention overcomes the drawbacks of the prior art and is directed to an improved ultrasonic surgical clamp coagulator shears apparatus that provides for a more ergonomic means for activating the shears by incorporating fingertip control on the handles.

### Brief Summary of the Invention

The present invention provides an instrument as defined in independent claim 1, with preferred embodiments provided in the appended claims.

An ultrasonic surgical clamp coagulator apparatus embodying the principles of the present invention is configured to permit hand activation for cutting, coagulation, and clamping of tissue during surgical procedures. In order to promote convenient and efficient use of the apparatus, the fingertip controls are provided directly into the disposal shears handle in a position that allows surgeons to activate the device without repositioning their hand. The two buttons provide independent control of the two power levels available from the generator, matching the two-foot pedal configuration.

In one embodiment the buttons are a rocker style configuration where the buttons appear independent to the user, but are actually a single unit rotating about a central axis. This configuration eliminates dual activation of the buttons, which would cause an error condition at the generator. The buttons are also incorporated in a manner in which the angle of depression/activation is not parallel, but rather, angled toward a common point in space to improve ergonomic feel. Further, the buttons are spaced to allow the user to rest their trigger finger (e.g. the index finger) between the buttons. This configuration minimizes the opportunity of an inadvertent activation and provides a high degree of grip stability during grasping and manipulation of tissue. In combination with the rocker switch are dome switches integrated within a flex circuit to provide for the electrical contact and snap feel of the rocker switches.

The invention further provides for an integrated electrical interface to the hand piece. A slip contact provides the required electrical interface between the shears and hand piece once the hand piece is securely mounted to the disposable shears device. In one embodiment, the electrical interface requires only two leads for control of both power levels.

In accordance with the illustrated embodiment, the present ultrasonic surgical clamp apparatus includes a housing that includes a handgrip portion. The apparatus further includes an elongated portion (which may be configured for endoscopic use), and a distal end positionable at the region at which tissue cutting, coagulation, and/or clamping is to be effected. In the preferred embodiment, two switches are mounted on the pistol grip for effecting activation of the generator to provide ultrasonic energy to the end-effector.

The present apparatus includes a clamping mechanism for clamping tissue against the ultrasonic end-effector. The clamping mechanism includes a clamp arm pivotally mounted on the distal end of the outer tubular sheath for pivotal movement with respect to the end-effector. Tissue is clamped between the clamp arm and the end-effector, thereby ultrasonically coupling the tissue with the end-effector (when energized) or permitting grasping and clamping of tissue when ultrasonic energy is not being transmitted through the waveguide to the end-effector. The clamp arm is operatively connected to the reciprocal actuating member of the apparatus so that reciprocal movement of the actuating member pivotally moves the clamp arm with respect to the end-effector.

An operating lever pivotally connected on the apparatus housing provides selective operation of the apparatus clamping mechanism. In the preferred embodiment, the operating lever, and associated handgrip portion of the housing are provided with a pistol-like configuration, thus permitting convenient movement of the operating lever by a user's thumb. The operating lever is interconnected with the reciprocal actuating member by a clamp drive mechanism so that pivotal movement of the operating lever reciprocally moves the actuating member for pivotally moving the clamp arm of the apparatus. Notably, the handgrip portion includes two pushbuttons for activating the end-effector, thus permitting the end-effector to be selectively activated by the surgeon's fingertip.

Further features and advantages of the present invention will become readily apparent from the following detailed description, the accompanying drawings, and the appended claims.

### Brief Description of the Figures

FIGURE 1 is a perspective view of an ultrasonic surgical system including an ultrasonic clamp coagulator apparatus embodying the principles of the present invention;
FIGURE 2 is an enlarged, elevation view fragmentary perspective view of a clamp mechanism of the clamp coagulator apparatus of Fig. 1;
FIGURE 3 is a side elevation, partially cut-away view, of the clamp coagulator embodying the principles of the present invention;
FIGURE 4 is an assembly drawing of a clamp coagulator of the present invention;
FIGURE 5 is an exploded view of the handle incorporating the rocker switch, handpiece connector, two slip rings and flex circuit;
FIGURES 6a-b are exploded views of the large slip ring and small slip ring, respectively;
FIGURE 7 is an exploded view of the handpiece connector;
FIGURE 8a-b is an exploded view of the flex circuit apparatus and the associated electrical schematic, respectively;
FIGURE 9 is a schematic view of the handle of an ultrasonic instrument of the present invention illustrating dimensional placement of the switches; and
FIGURE 10 is an exploded view of the switch assembly.

### Detailed Description of the Invention

Before explaining the present invention in detail, it should be noted that the invention is not limited in its application or use to the details of construction and arrangement of parts illustrated in the accompanying drawings and description. The illustrative embodiments of the invention may be implemented or incorporated in other embodiments, variations and modifications, and may be practiced or carried out in various ways. Furthermore, unless otherwise indicated, the terms and expressions employed herein have been chosen for the purpose of describing the illustrative embodiments of the present invention for the convenience of the reader and are not for the purpose of limiting the invention. The scope of the present invention is defined in the appended claims.

The present invention is particularly directed to an improved ultrasonic surgical clamp coagulator apparatus which is configured for effecting tissue cutting, coagulation, and/or clamping during surgical procedures. The present apparatus can readily be configured for use in both open surgical procedures, as well as laparoscopic or endoscopic procedures. Versatile use is facilitated by selective use of ultrasonic energy. When ultrasonic components of the apparatus are inactive, tissue can be readily gripped and manipulated, as desired, without tissue cutting or damage. When the ultrasonic components are activated, the apparatus permits tissue to be gripped for coupling with the ultrasonic energy to effect tissue coagulation, with application of increased pressure efficiently effecting tissue cutting and coagulation. If desired, ultrasonic energy can be applied to tissue without use of the clamping mechanism of the apparatus by appropriate manipulation of the ultrasonic "blade" or end-effector of the device.

As will become apparent from the following description, the present clamp coagulator apparatus is particularly configured for disposable use by virtue of its straightforward construction. As such, it is contemplated that the apparatus be used in association with an ultrasonic drive unit of a surgical system, whereby ultrasonic energy from the drive unit provides the desired ultrasonic actuation of the present clamp coagulator apparatus. It will be appreciated that a clamp coagulator apparatus embodying the principles of the present invention can be configured for non-disposable use, and non-detachably integrated with an associated ultrasonic drive unit. However, detachable connection of the present clamp coagulator apparatus with an associated ultrasonic drive unit is presently preferred for single-patient use of the apparatus.

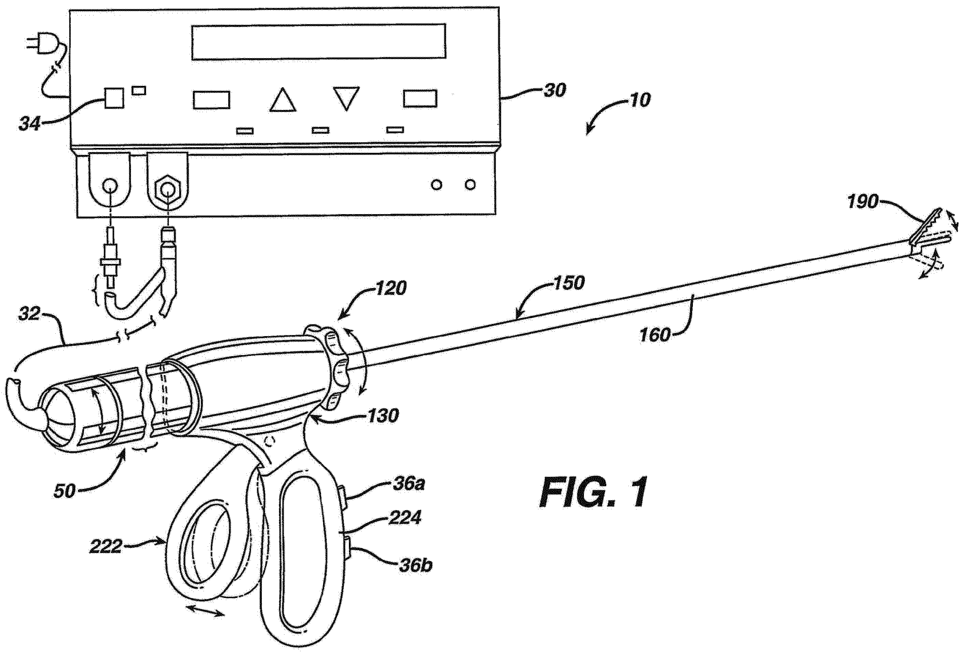

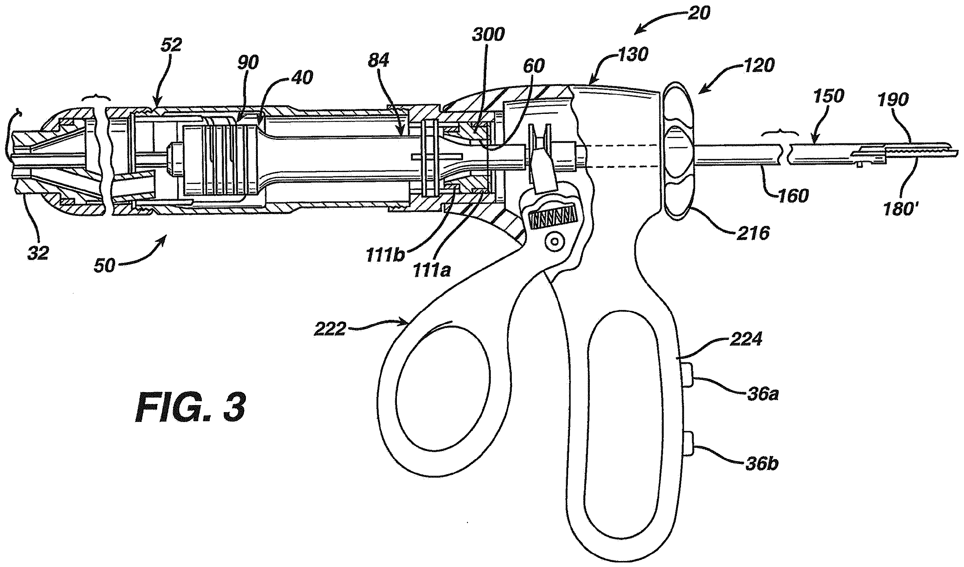

With reference first to FIGS. 1 and 3, therein is illustrated a presently preferred embodiment of a surgical system, generally designated 10, which includes an ultrasonic clamp coagulator apparatus embodying the principles of the present invention. Preferred details of the ultrasonic generator and associated ultrasonic drive unit of the surgical system 10 will first be described, with subsequent detailed description of the fingertip activation of the end-effector, embodying the principles of the present invention.

The surgical system 10 includes an ultrasonic generator 30 and an associated ultrasonic surgical instrument. The surgical instrument includes an ultrasonic drive unit, designated 50, and an ultrasonic clamp coagulator apparatus 120 embodying the principles of the present invention. As will be further described, an ultrasonic transducer of the drive unit 50, and an ultrasonic waveguide of the clamp coagulator 120, together provides an acoustic assembly of the present surgical system, with the acoustic assembly providing ultrasonic energy for surgical procedures when powered by generator 30. It will be noted that in some applications, the ultrasonic drive unit 50 is referred to as a "hand piece assembly" because the surgical instrument of the surgical system is configured such that a surgeon grasps and manipulates the ultrasonic drive unit 50 during various procedures and operations. The clamp coagulator apparatus 120 embodying the principles of the present invention preferably includes a pistol-like grip arrangement that facilitates positioning and manipulation of the instrument apart from manipulation of the ultrasonic drive unit 50.

The generator 30, for example, a Generator 300 available from Ethicon Endo-Surgery, Inc., Cincinnati, Ohio, of the surgical system sends an electrical signal through a cable 32 at a selected current, frequency, and phase determined by a control system of the generator 30. As will be further described, the signal causes one or more piezoelectric elements of the acoustic assembly of the surgical instrument to expand and contract, thereby converting the electrical energy into mechanical motion. The mechanical motion results in longitudinal waves of ultrasonic energy that propagate through the acoustic assembly in an acoustic standing wave to vibrate the acoustic assembly at a selected frequency and excursion. An end-effector at the distal end of the waveguide of the acoustic assembly is placed in contact with tissue of the patient to transfer the ultrasonic energy to the tissue. As further described below, a surgical tool, such as, a jaw or clamping mechanism, is preferably utilized to press the tissue against the end-effector.

As the end-effector couples with the tissue, thermal energy or heat is generated as a result of friction, acoustic absorption, and viscous losses within the tissue. The heat is sufficient to break protein hydrogen bonds, causing the highly structured protein (i.e., collagen and muscle protein) to denature (i.e., become less organized). As the proteins are denatured, a sticky coagulum forms to seal or coagulate small blood vessels. Deep coagulation of larger blood vessels results when the effect is prolonged.

The transfer of the ultrasonic energy to the tissue causes other effects including mechanical tearing, cutting, cavitation, cell disruption, and emulsification. The amount of cutting as well as the degree of coagulation obtained varies with the excursion of the end-effector, the frequency of vibration, the amount of pressure applied by the user, the sharpness of the end-effector, and the coupling between the end-effector and the tissue.

As illustrated in FIGs. 1 and 3, the generator 30 includes a control system integral with the generator 30 and an on-off switch 34. The power switch 34 controls the electrical power to the generator 30, and when activated by the triggering mechanism 36a-b, the generator 30 provides energy to drive the acoustic assembly 40 of the surgical system 10 to drive the end-effector at a predetermined excursion level. The generator 30 drives or excites the acoustic assembly at any suitable resonant frequency of the acoustic assembly.

When the generator 30 is activated via the triggering mechanism 36a-b, the generator 30 continuously applies electrical energy to a transducer stack 90. A phase-locked loop in the control system of the generator 30 monitors feedback from the acoustic assembly. The phase lock loop adjusts the frequency of the electrical energy sent by the generator 30 to match the resonant frequency of the selected longitudinal mode of vibration of the acoustic assembly including the tissue load. In addition, a second feedback loop in the control system maintains the electrical current supplied to the acoustic assembly at a preselected constant level in order to achieve substantially constant excursion at the end-effector of the acoustic assembly.

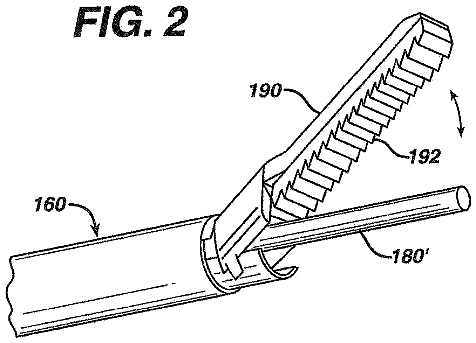

The electrical signal supplied to the acoustic assembly will cause the distal end of the waveguide, i.e., the end-effector, (FIG. 2) to vibrate longitudinally in the range of, for example, approximately 20 kHz to 250 kHz, and preferably in the range of about 54 kHz to 56 kHz, and most preferably at about 55.5 kHz. The excursion of the vibrations at the end-effector can be controlled by, for example, controlling the amplitude of the electrical signal applied to the transducer assembly 40 by the generator 30. Switch 36a provides for one level of amplitude and switch 36b provides for a second level of amplitude.

As noted above, the triggering mechanism 36a-b of the generator 30 allows a user to activate the generator 30 so that electrical energy may be continuously supplied to the acoustic assembly. The triggering mechanism 36a-b preferably comprises a rocker switch that is positioned on handle 224 and electrically coupled or attached to the generator 30 by a cable or cord. Alternatively, the triggering mechanism 36a-b could be placed at other convenient locations, for example, on thumb ring 222 or on shroud 130.

Referring to FIGS. 1, 3 and 4, the handpiece 50 includes a multi-piece housing 52 adapted to isolate the operator from the vibrations of the acoustic assembly. The drive unit housing 52 can be shaped to be held by a user in a conventional manner, but it is contemplated that the present clamp coagulator 120 principally be grasped and manipulated by a pistol-like arrangement provided by a housing of the apparatus, as will be described. While the multi-piece housing 52 is illustrated, the housing 52 may comprise a single or unitary component.

The housing 52 generally includes a proximal end, a distal end, and a cavity extending longitudinally therein. The distal end of the housing 52 includes an opening 60 configured to allow the acoustic assembly of the surgical system 10 to extend therethrough, and the proximal end of the housing 52 is coupled to the generator 30 by the cable 32.

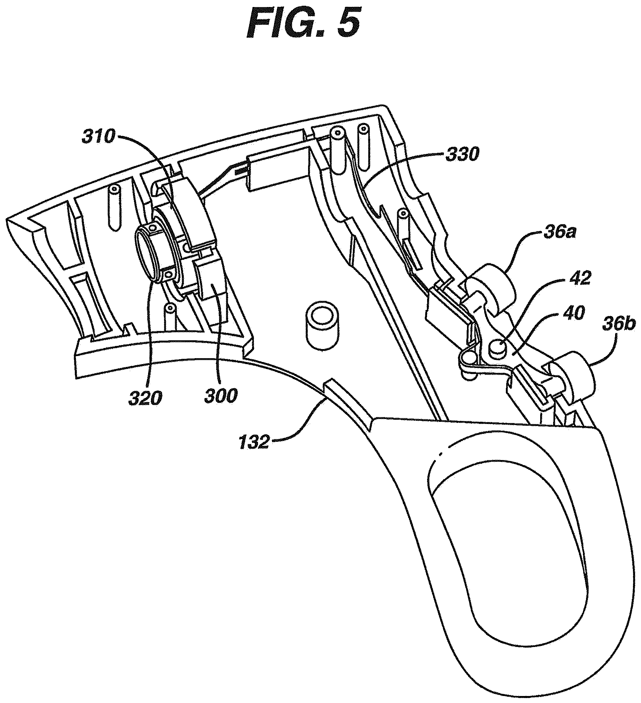

The housing 52 is preferably constructed from a aluminum, however, it is also contemplated that housing 52 may be made from a variety of plastics, such as Ultem RTM. A suitable ultrasonic drive unit 50 is model no. HP054, available from Ethicon Endo-Surgery, Inc., Cincinnati, Ohio. Two gold-plated circumferential electrical connectors 111a and 111b are located at the distal end of drive unit 50 for communicating electrical control signals from switches 36a-b to the generator 30. As shown in FIGS. 3 and 5, the handpiece 50 is preferably acoustically coupled to the second acoustic portion of the ultrasonic clamp coagulator apparatus 120. The distal end of the drive unit 50 is preferably coupled to the proximal end of the second acoustic portion by an internal threaded connection near an anti-node, but alternative coupling arrangements can be employed. When drive unit 50 is inserted into housing 130 and connected thereto, the distal end of drive unit 50 passes through connector 300 and ring connectors 111a-b interface with slip ring connectors 310 and 320, respectively, as is discussed in more detail below.

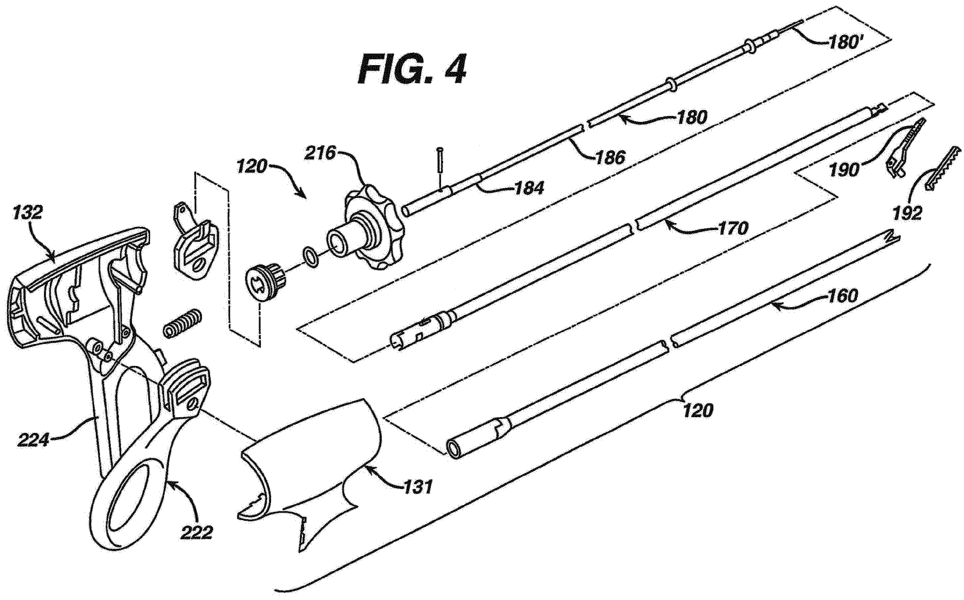

Referring also now to FIG. 4, an exploded view of the ultrasonic clamp coagulator apparatus 120 of the surgical system 10 in accordance with a preferred embodiment is illustrated. The proximal end of the ultrasonic clamp coagulator apparatus 120 preferably receives and is fitted to the distal end of the ultrasonic drive unit 50 by insertion of the drive unit into the housing of the apparatus, as shown in FIG. 3. The ultrasonic clamp coagulator apparatus 120 is preferably attached to and removed from the ultrasonic drive unit 50 as a unit. The ultrasonic clamp coagulator 120 may be disposed of after a single use.

The ultrasonic clamp coagulator apparatus 120 preferably includes a handle assembly or a housing 130, preferably comprising mating housing portions 131, 132, and an elongated or endoscopic portion 150. When the present apparatus is configured for endoscopic use, the construction can be 5 dimensioned such that portion 150 has an outside diameter of about 5.5 mm. The elongated portion 150 of the ultrasonic clamp coagulator apparatus 120 extends orthogonally from the apparatus housing 130. The elongated portion 150 can be selectively rotated with respect to the housing 130. The elongated portion 150 preferably includes an outer tubular member or sheath 160, an inner tubular actuating member 170, and the second acoustic portion of the acoustic system in the form of a waveguide 180 having an end-effector 180'. Outer tube 160, inner tube 170, end effector 180' and clamp pad 190 are all operatively coupled with rotation knob 216 so that rotation of knob 216 causes corresponding rotation of the end effector 180' and clamp arm 190. As illustrated in FIG. 4, the proximal end of the waveguide 180 of the second acoustic portion is preferably detachably coupled to the mounting device 84 of the ultrasonic drive unit 50 near an anti-node as described above. The waveguide 180 preferably has a length substantially equal to an integer number of one-half system wavelengths. The waveguide 180 is preferably fabricated from a solid core shaft constructed out of material that propagates ultrasonic energy efficiently, such as titanium alloy (i.e., Ti-6AI-4V) or an aluminum alloy. It is contemplated that the waveguide 180 can alternatively be fabricated from any other suitable material.

With particular reference to FIG. 2, a clamping mechanism of the present clamp coagulator 120 is configured for cooperative action with the end-effector 180' of the waveguide 180. The clamping mechanism includes a pivotally movable clamp arm 190, which is pivotally connected at the distal end thereof to the distal end of outer tubular sheath 160. A clamp pad 192, preferably formed from Teflon or other suitable low-friction material, is mounted on the surface of the clamp arm for cooperation with the end-effector 180', with pivotal movement of the clamp arm positioning the clamp pad in substantially parallel relationship to, and in contact with, the end-effector 180'. By this construction, tissue is grasped between the pad 192 and the end effector 180'. As illustrated, the pad 192 is preferably provided with a saw tooth-like configuration to enhance the gripping of tissue in cooperation with the end-effector 180'. As will be appreciated by those skilled in the art, end-effector 180' and clamp pad 190 may take on any number of shapes, including a curved shaped as disclosed in U.S. Patent No. 6,325,811.

As is described in the U.S. patents previously referred to, the surgeon's thumb squeezes trigger 222 to cause the clamping mechanism to pivot the movable clamp arm 190. One or more of the surgeon's other fingers may rest comfortably within handle 224. In accordance with the current invention, the surgeon's index finger controls the operation of the generator 30 by selectively depressing switches 36a-b. Switches 36a-b are conveniently located such that the surgeon may energize end effector 180' and also cause rotation of the end effector 180' and clamp pad 190 via knob 216 using the same hand (fingers) for operation.

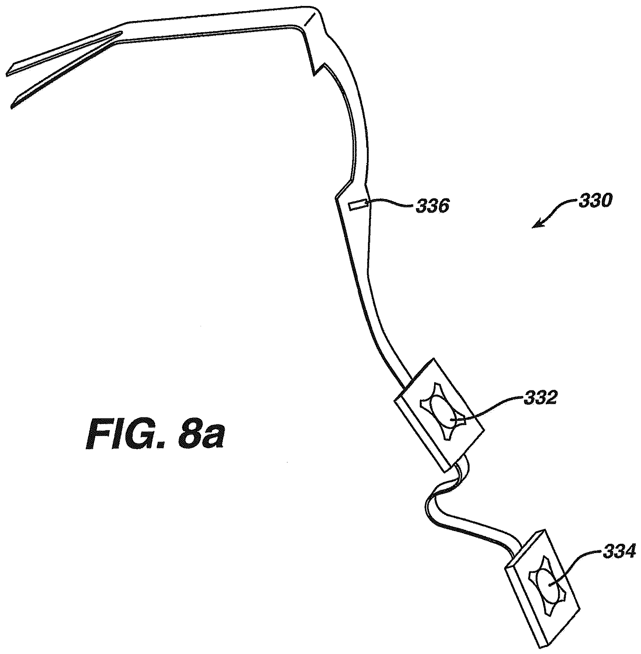

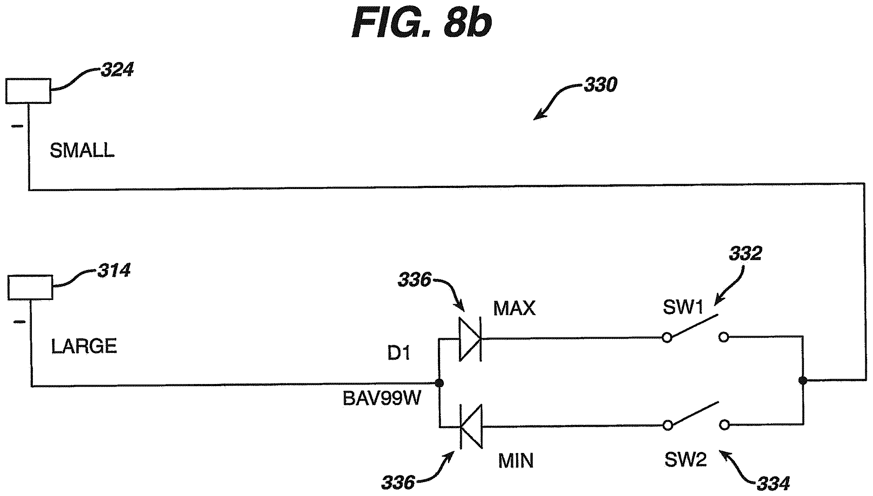

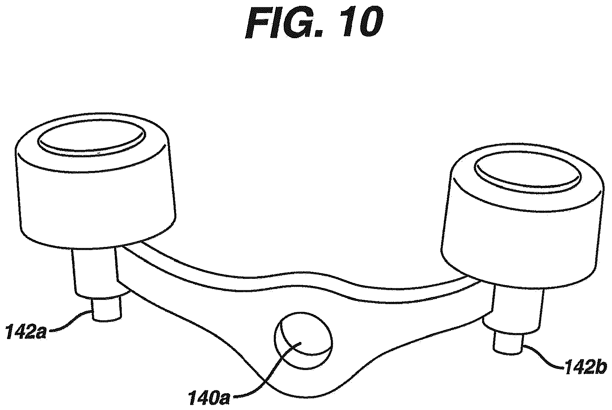

Referring now to Figs. 5-8 and 10, switches 36a-b are mechanically connected via a rocker arm 40 comprising an aperture 140a for accepting pivot post 42. In this configuration, switches 36a-b cannot be simultaneously depressed, which, if were the case, would provide an error message from generator 30. A flex circuit 330 provides for the electro-mechanical interface between switches 36a-b and the generator 30 via the drive unit 50. Also referring to Fig. 8a, flex circuit 330 includes, at the distal end, two dome switches 332 and 334 that are mechanically actuated by depressing pins 142a-b of corresponding switches 36a-b, respectively. Dome switches 332 and 334 are electrical contact switches, that when depressed provide an electrical signal to generator 30 as shown by the electrical wiring schematic of Fig. 8b. Flex circuit 330 also comprises two diodes within a diode package 336, also illustrated in Fig. 8b. Flex circuit 330 provides conductors, as is known to those in the art, that connect to slip ring conductors 310 and 320 via connector 300, which in turn provide electrical contact to ring conductors 111a-b, which in turn are connected to conductors in cable 32 that connect to generator 30. Ring conductors 111a-b are situated within the distal end of handpiece 50 as is generally described in U.S. Patent No. 6,623,500 B1.

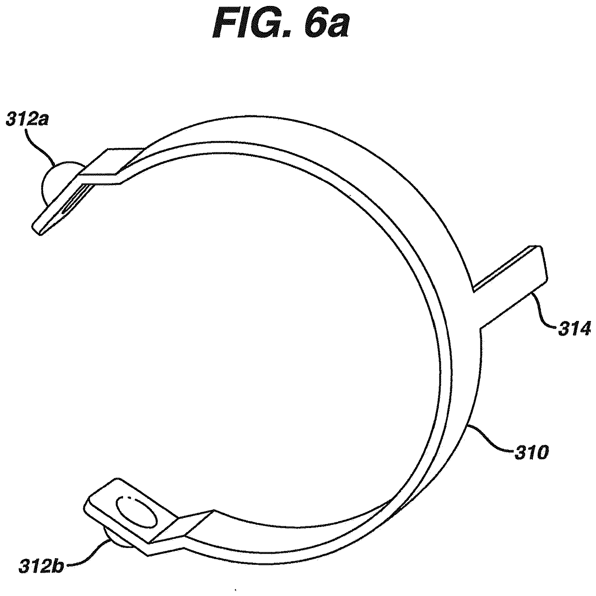

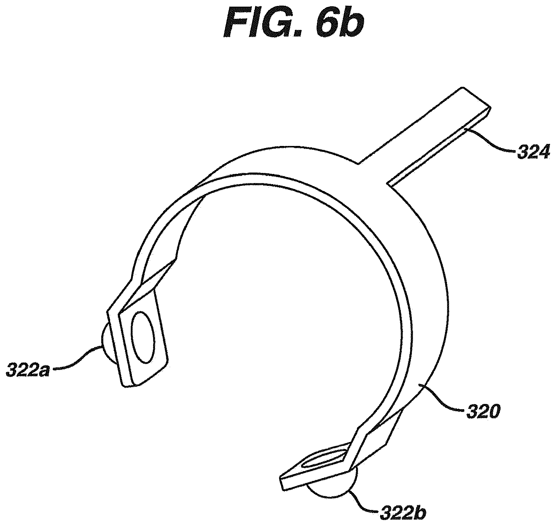

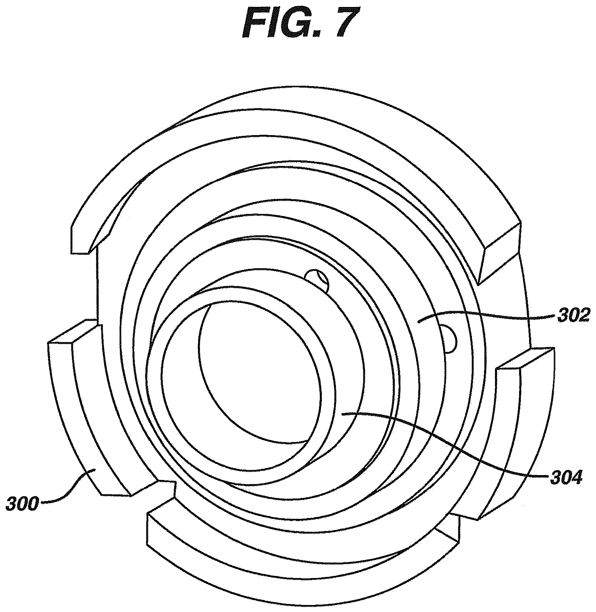

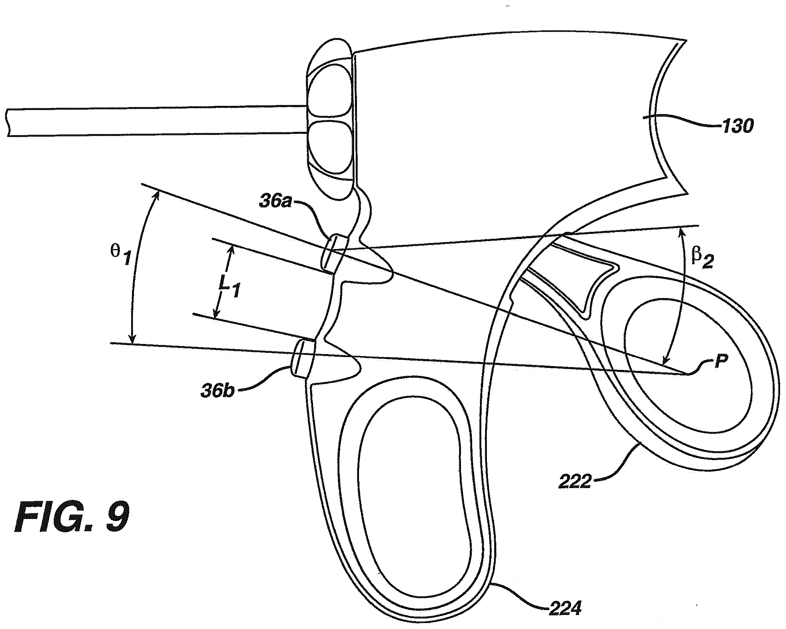

With particular reference now to Figs. 6a-b and 7, slip ring conductors 310 and 320 are generally open-ended O-shaped springs that slip onto mounting surfaces 302 and 304 of connector 300, respectively. Each spring slip-ring comprises two pressure point contacts (312a-b and 322a-b) that contact the respective ring conductor 111a-b of handpiece 50. The spring tension of the slip rings 310 and 320 cause positive contact between contacts 312a-b, 322a-b and conductors 111a-b. It is evident that the slip-ring construction allows electrical contact to be made even as hand piece 50 may be rotated by the surgeon during use of the instrument. Posts 314 and 324 of the respective slip rings electrically connect to the respective conductor within flex circuit 330 to complete the electrical circuit as shown in Fig. 8b. .Referring now to Fig. 9, switches 36a-b are preferably configured in such a way to provide an ergonomically pleasing grip and operation for the surgeon. In particular, the angle of depression/activation of switches 36a-b is not parallel, but the direction of activation for each switch define an angle of actuation θ₁ with respect to a common point P within area of thumb placement of the thumb grip of trigger 222, when the trigger 222 is in its normal state. The range of angle θ₁is from about 10° to about 30°, and more preferably from about 15° to about 20°. Switches 36a-b are also separated by a distance L₁, which is sufficient to minimize inadvertent activation by the surgeon's finger resting on handle 224 between switches 36a-b, but at the same time provides for a high degree of grip stability during tissue grasping and manipulation functions. Distance L₁ is from about 2.54 cm (1 inch) to about 1.27 cm (0.5 inches), and more preferably, from about 2.03 cm (0.8 inches) to about 1.52 cm (0.6 inches).

While the present invention has been illustrated by description of several embodiments, it is not the intention of the applicant to restrict or limit the scope of the appended claims to such detail. Numerous variations, changes, and substitutions will occur to those skilled in the art without departing from thescope of the invention. Moreover, the structure of each element associated with the present invention can be alternatively described as a means for providing the function performed by the element. Accordingly, it is intended that the invention be limited only by the scope of the appended claims.

## Claims

1. An ultrasonic surgical instrument that includes an ultrasonic drive unit (50) comprising an ultrasonic transducer (90) for converting electrical energy to mechanical motion resulting in ultrasonic energy, the ultrasonic surgical instrument comprising:
a housing (130) having a handle (224) configured to interface with a user of the instrument and a first end for accepting the ultrasonic drive unit (50), the housing comprising:
an elongated portion (150) comprising an outer tubular sheath (160) extending orthogonally from the housing (130) and having an ultrasonic waveguide (180) positioned within the elongated portion, and positioned within and extending distally from a second end of the housing (130), the waveguide having an end effector (180') with a clamp arm (190), wherein the clamp arm (190) is pivotally mounted on a distal end of the outer tubular sheath (160) and a proximal end of the waveguide (180) being detachably coupled to the drive unit (50);
a rotation knob (216) operatively coupled to the second end of the housing for causing rotation of the elongated portion, waveguide (180), end effector (180') and clamp arm (190); and **characterized by** at least one switch (36) located on the handle (224);
the switch is electrically connected to the drive unit (50) via a circumferential electrical connector (111a, b) on the drive unit for providing an electrical signal from the at least one switch (36) to a generator for controlling the level of ultrasonic energy delivered by the ultrasonic transducer (90).

2. The ultrasonic surgical instrument of claim 1, wherein the handle (224) comprises two switches (36), the first switch electrically connected to the drive unit (50) for providing a first electrical signal from the first switch to the generator, and the second switch electrically connected to the drive unit (50) for providing a second electrical signal from the second switch to the generator.

3. The ultrasonic instrument of claim 2, wherein the two switches (36) are separated by a distance from about twelve and a half millimetres to about twenty five millimetres (one-half inch to about one inch).

4. The ultrasonic instrument of claim 2, wherein the first and second switches (36) each define a line of actuation that form an angle of actuation from about 10° to about 30°.

## Patentansprüche

1. Chirurgisches Ultraschallinstrument, das eine Ultraschallantriebseinheit (50) umfasst, die einen Ultraschallwandler (90) zum Umwandeln von elektrischer Energie in mechanische Bewegung umfasst, die in Ultraschallenergie resultiert, wobei das chirurgische Ultraschallinstrument umfasst:
ein Gehäuse (130) mit einem Griff (224), der dazu ausgelegt ist, eine Schnittstelle zu einem Benutzer des Instruments zu bilden, und ein erstes Ende zum Aufnehmen der Ultraschallantriebseinheit (50), wobei das Gehäuse umfasst:
einen länglichen Abschnitt (150), der eine röhrenförmige Außenhülle (160) umfasst, die sich orthogonal von dem Gehäuse (130) erstreckt und einen Ultraschallwellenleiter (180) aufweist, der innerhalb des länglichen Abschnitts positioniert ist, und in einem zweiten Ende des Gehäuses (130) positioniert ist und sich distal davon erstreckt, wobei der Wellenleiter einen Endeffektor (180') mit einem Klemmarm (190) aufweist, wobei der Klemmarm (190) schwenkbar an einem distalen Ende der röhrenförmigen Außenhülle (160) montiert ist und ein proximales Ende des Wellenleiters (180) lösbar an der Antriebseinheit (50) gekoppelt ist;
einen Drehknopf (216), der funktionell mit dem zweiten Ende des Gehäuses gekoppelt ist, um eine Drehung des länglichen Abschnitts, des Wellenleiters (180), des Endeffektors (180') und des Klemmarms (190) zu bewirken; und
**gekennzeichnet durch** mindestens einen Schalter (36), der sich an dem Griff (224) befindet;
wobei der Schalter über einen elektrischen umlaufenden Verbinder (111a, b) an der Antriebseinheit elektrisch mit der Antriebseinheit (50) verbunden ist, zum Bereitstellen eines elektrischen Signals von dem mindestens einen Schalter (36) für den Generator zum Steuern des Niveaus der von dem Ultraschallwandler (90) gelieferten Ultraschallenergie.

2. Chirurgisches Ultraschallinstrument nach Anspruch 1, wobei der Griff (224) zwei Schalter (36) umfasst, wobei der erste Schalter elektrisch mit der Antriebseinheit (50) verbunden ist, zum Bereitstellen eines ersten elektrischen Signals von dem ersten Schalter für den Generator, und wobei der zweite Schalter elektrisch mit der Antriebseinheit (50) verbunden ist, zum Bereitstellen eines zweiten elektrischen Signals von dem zweiten Schalter für den Generator.

3. Ultraschallinstrument nach Anspruch 2, wobei die zwei Schalter (36) um einen Abstand von etwa zwölfeinhalb Millimeter bis etwa fünfundzwanzig Millimeter (einem halben Zoll bis einem Zoll) getrennt sind.

4. Ultraschallinstrument nach Anspruch 2, wobei der erste und der zweite Schalter (36) jeweils eine Betätigungsleitung definieren, die einen Betätigungswinkel von ungefähr 10° bis ungefähr 30° bilden.

## Revendications

1. Instrument chirurgical ultrasonore qui comprend une unité d'entraînement ultrasonore (50) comprenant un transducteur ultrasonore (90) pour convertir une énergie électrique en mouvement mécanique, ce qui permet d'obtenir une énergie ultrasonore, l'instrument chirurgical ultrasonore comprenant :
un boîtier (130) comportant une poignée (224) conçue pour assurer l'interface avec un utilisateur de l'instrument et une première extrémité pour accepter l'unité d'entraînement ultrasonore (50), le boîtier comprenant :
une partie allongée (150) comprenant une gaine tubulaire externe (160) s'étendant orthogonalement depuis le boîtier (130) et possédant un guide d'onde ultrasonore (180) positionné à l'intérieur de la partie allongée, et positionné à l'intérieur d'une seconde extrémité du boîtier (130) et s'étendant distalement depuis celle-ci, le guide d'onde comportant un effecteur terminal (180') assorti d'un bras de serrage (190), dans lequel le bras de serrage (190) est monté pivotant sur une extrémité distale de la gaine tubulaire externe (160) et une extrémité proximale du guide d'onde (180) pouvant être reliée de manière détachable à l'unité d'entraînement (50) ;
un bouton de rotation (216) relié fonctionnellement à la seconde extrémité du boîtier pour provoquer une rotation de la partie allongée, du guide d'onde (180), de l'effecteur terminal (180') et du bras de serrage (190) ; et
**caractérisé par** au moins un commutateur (36) situé sur la poignée (224) ;
le commutateur est connecté électriquement à l'unité d'entraînement (50) par l'intermédiaire d'un connecteur électrique circonférentiel (111a, b) sur l'unité d'entraînement pour fournir un signal électrique de l'au moins un commutateur (36) à un générateur pour commander le niveau d'énergie ultrasonore délivrée par le transducteur ultrasonore (90) .

2. Instrument chirurgical ultrasonore selon la revendication 1, dans lequel la poignée (224) comprend deux commutateurs (36), le premier commutateur étant connecté électriquement à l'unité d'entraînement (50) pour fournir un premier signal électrique du premier commutateur au générateur, et le second commutateur connecté électriquement à l'unité d'entraînement (50) pour fournir un second signal électrique du second commutateur au générateur.

3. Instrument ultrasonore selon la revendication 2, dans lequel les deux commutateurs (36) sont séparés par une distance d'environ douze millimètres et demi à environ vingt-cinq millimètres (un demi-pouce à environ un pouce) .

4. Instrument ultrasonore selon la revendication 2, dans lequel les premier et second commutateurs (36) définissent chacun une ligne d'actionnement formant un angle d'actionnement d'environ 10° à environ 30°.
